(19) Europäisches Patentamt
European Patent Office
Office européen des brevets

(11) **EP 3 590 562 B1**

(12) **EUROPEAN PATENT SPECIFICATION**

(45) Date of publication and mention
of the grant of the patent:
**07.04.2021 Bulletin 2021/14**

(51) Int Cl.:
***A61M 1/36*** *(2006.01)*    ***A61M 1/34*** *(2006.01)*

(21) Application number: **18760556.3**

(22) Date of filing: **22.02.2018**

(86) International application number:
**PCT/JP2018/006462**

(87) International publication number:
**WO 2018/159451 (07.09.2018 Gazette 2018/36)**

(54) **BLOOD PURIFICATION DEVICE**

BLUTREINIGUNGSVORRICHTUNG

DISPOSITIF DE PURIFICATION DU SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.03.2017 JP 2017039749**

(43) Date of publication of application:
**08.01.2020 Bulletin 2020/02**

(73) Proprietor: **Asahi Kasei Medical Co., Ltd.
Tokyo 1000006 (JP)**

(72) Inventors:
• **UMIMOTO, Takashi
60528 Frankfurt am Main (DE)**
• **DOSIL ROSENDE Pablo
36600 Vilagarcia de Arousa Pontevendra (ES)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
WO-A1-2016/172238    JP-A- 2002 248 165
JP-A- 2009 509 584    JP-A- 2009 527 343
JP-A- 2014 511 211    JP-A- 2016 087 438
US-A- 4 500 309    US-A1- 2002 107 469
US-A1- 2011 168 614    US-A1- 2011 168 614
US-A1- 2011 208 105    US-A1- 2014 353 251
US-B2- 9 039 647

## Description

## Technical Field

The present invention relates to a blood purification apparatus.

## Background Art

Blood purification apparatuses are typically used for blood purification treatment performed in dialysis therapy, plasma exchange therapy, and the like. Such blood purification apparatus is comprised of a blood purifier and a blood circuit for feeding blood collected from a patient to the blood purifier and then returning the purified blood from the blood purifier back to the patient.

In order to prevent coagulation of the patient's blood in the blood circuit, an anticoagulant is conventionally used by introducing the anticoagulant into the blood circuit at a portion upstream of the blood purifier.

While heparin is conventionally used as such anticoagulant, the use of citric acid has recently been developed. Citric acid places less burden on the blood purification membrane in the blood purifier and is also applicable to patients who have the tendency to bleed.

When citric acid is used as an anticoagulant, the amount of ionized calcium in the blood decreases with the effect of the citric acid, and the amount of Ci-Ca conjugates (a type of calcium complex) increases. Most of such calcium complexes are removed by the blood purification membrane of the blood purifier. As a result, the blood to be returned to the patient after the passage of the blood purifier has a significantly lowered calcium concentration. For this reason, in a blood purification apparatus, a device for adding calcium is provided in the blood circuit downstream of the blood purifier (see Patent Document 1).

US 9,039,647 discloses a blood purification apparatus with regional citrate anticoagulation, wherein the flow rate V of calcium solution to be supplied to the blood circuit is calculated such that the level of total calcium TCAretum of blood returned to the patient is equal to the level of total calcium TCA of blood entering the extracorporeal blood circuit.. TCA is thus a target value (for TCAretum). The low rate V is calculated on the basis of an estimated amount of calcium loss throughout the blood circuit, which is calculated on the basis of TCA and on the amount of ionized calcium B in the patient's blood.

US 2014/353251 discloses a blood purification apparatus with a regional citrate anticoagulated extracorporeal blood circuit comprising a blood purifier, wherein the patient's blood total calcium level and calcium ion level is predicted on the basis of a mathematical model. The mathematical model makes use of a target for the calcium ion level of the patient's blood to control the regional citrate anticoagulation, but the calcium solution flow rate to be supplied is based on an amount of calcium loss throughout the blood circuit that is not estimated on the basis of the target for the calcium ion level but on the current prediction of the patient's ionized and total calcium levels.

Citation List

Patent Document

Patent Document 1: US2016/0121035

## Summary

Technical Problem

However, in order to determine the flow rate of calcium to be added to the patient's blood in the blood circuit, the above-described blood purification apparatus needs to sample the patient's blood in the blood circuit to determine its calcium concentration. This leads to a loss of the patient's blood and puts strain on the patient. Furthermore, this is a burdensome process for medical service workers.

The present invention has been made under the above circumstances, and an object of the present invention is to provide a blood purification apparatus capable of adjusting the calcium concentration in a patient's blood in a simple and automatic manner during the purification of the patient's blood.

Solution to Problem

The present inventors have found, as a result of intensive studies, that the above object can be achieved by estimating a loss of calcium occurring in the entire blood circuit based on a target calcium ion concentration for a patient's blood and then calculating the flow rate of calcium solution to be supplied to the blood circuit based on the estimated calcium loss. With this finding, the present inventors have completed the present invention.

That is, the present invention includes the following aspects.

(1) A blood purification apparatus comprising: a blood circuit; a blood purifier provided in the blood circuit; citric acid solution supply means that supplies a citric acid solution into the blood circuit upstream of the blood purifier; and calcium solution supply means that supplies a calcium solution into the blood circuit downstream of the blood purifier, wherein the blood purification apparatus is configured to: estimate, based on an input of a target calcium ion concentration for a patient's blood, an amount of calcium loss throughout the blood circuit, including an amount of calcium removed by the blood purifier when blood having the target calcium ion concentration flows through the blood circuit; calculate a flow rate of the calcium solution to be supplied to the blood circuit based on the estimated amount of calcium loss; and supply the calcium solution to the blood circuit by the calcium solution supply means based on the calculated flow rate of the calcium solution.

(2) The blood purification apparatus as set forth in (1), wherein the flow rate of the calcium solution (Qca) to be supplied to the blood circuit by the calcium solution supply means is calculated according to Qca = {{(Qd*kd+Qf*kf)*Ctca*kca-Qr*Crca}/Cica}, wherein: Ctca is a target calcium ion concentration for the patient's blood; Cica is a concentration of the calcium solution in the calcium solution supply means; kca is a constant used for calculating a concentration of calcium that can be subjected to dialysis and/or filtration at the blood purifier based on the target calcium ion concentration Ctca; Qd is a flow rate of dialysate passing through the blood purifier; kd is a constant used for calculating an amount of calcium removed by dialysis based on the flow rate of dialysate Qd; Qf is a flow rate of filtration at the blood purifier; kf is a constant used for calculating an amount of calcium removed by filtration based on the flow rate of filtration Qf; Qr is a flow rate of a replacement fluid added to the blood circuit; and Crca is a calcium concentration of the replacement fluid.

(3) The blood purification apparatus as set forth in (1), wherein the flow rate of the calcium solution (Qca) to be supplied to the blood circuit by the calcium solution supply means is calculated according to Qca = {{(Qd*kd+Qf*kf)*Ctca*kca}/Cica}, wherein: Ctca is a target calcium ion concentration for the patient's blood; Cica is a concentration of the calcium solution in the calcium solution supply means; kca is a constant used for calculating a concentration of calcium that can be subjected to dialysis and/or filtration at the blood purifier based on the target calcium ion concentration Ctca; Qd is a flow rate of dialysate passing through the blood purifier; kd is a constant used for calculating an amount of calcium removed by dialysis based on the flow rate of dialysate Qd; Qf is a flow rate of filtration at the blood purifier; and kf is a constant used for calculating an amount of calcium removed by filtration based on the flow rate of filtration Qf.

(4) The blood purification apparatus as set forth in (1), wherein the flow rate of the calcium solution (Qca) to be supplied to the blood circuit by the calcium solution supply means is calculated according to Qca = {(Qf*kf*Ctca*kca-Qr*Crca}/Cica}, wherein: Ctca is a target calcium ion concentration for the patient's blood; Cica is a concentration of the calcium solution in the calcium solution supply means; kca is a constant used for calculating a concentration of calcium that can be subjected to filtration at the blood purifier based on the target calcium ion concentration Ctca; Qf is a flow rate of filtration at the blood purifier; kf is a constant used for calculating an amount of calcium removed by filtration based on the flow rate of filtration Qf; Qr is a flow rate of a replacement fluid added to the blood circuit; and Crca is a calcium concentration of the replacement fluid.

(5) The blood purification apparatus as set forth in any of (2) to (4), wherein a correction term can be set to correct the flow rate of the calcium solution to be supplied.

Advantageous Effects of Invention

According to the present invention, during the purification of a patient's blood, the calcium concentration in the blood can be adjusted in a simple and automatic manner.

**Brief Description of Drawings**

Fig. 1 is an illustration showing the schematic configuration of a blood purification apparatus according to a first embodiment.

Fig. 2 is a block diagram showing an example of the configuration of a control device.

Fig. 3 is a chart indicating the change of calcium ion concentration in a patient's blood.

Fig. 4 is an illustration showing the schematic configuration of a blood purification apparatus according to a second embodiment.

Fig. 5 is an illustration showing the schematic configuration of a blood purification apparatus according to a third embodiment.

**Description of Embodiments**

Hereinafter, preferred embodiments of the present invention will be described with reference to the attached drawings. It should be noted that the same elements will be given the same reference numerals and any repetitive explanation will be omitted. Furthermore, unless otherwise specified, the positional relationships, such as up/down and left/right relationships, are based on those shown in the drawings. In addition, the dimensional ratios shown in the drawings are not limited to those in the drawings. The below embodiments are merely examples for describing the present invention and the present invention is not limited to the described embodiments.

First Embodiment

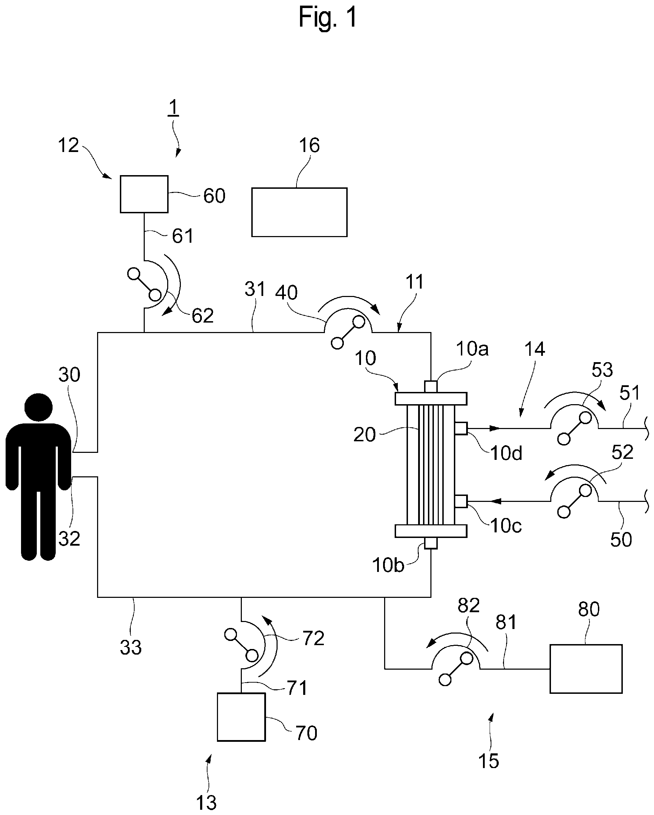

Fig. 1 is an illustration showing the schematic configuration of a blood purification apparatus 1 according to the present embodiment. The blood purification apparatus 1 of this embodiment is an apparatus for filtration and dialysis of blood (hemodiafiltration).

The blood purification apparatus 1 includes, for example, a blood purifier 10, a blood circuit 11, citric acid solution supply means 12, calcium solution supply means 13, a dialysate circuit 14, a replacement fluid circuit 15 and a control device 16.

The blood purifier 10 has a cylindrical module containing a blood purification membrane 20 formed of, for example, a hollow fiber membrane. The blood purifier 10 has liquid ports 10a and 10b that communicate with a primary side (blood side) of the blood purification membrane 20 and liquid ports 10c and 10d that communicate with a secondary side (dialysate side) of the blood purification membrane 20.

The blood circuit 11 includes, for example, a blood collection circuit 31 connecting a blood collection part 30 to the liquid port 10a of the blood purifier 10, and a blood return circuit 33 connecting the liquid port 10b of the blood purifier 10 to a blood return part 32. The blood circuit 11 is formed of a soft tube.

The blood collection circuit 31 is provided with, for example, a blood pump 40. Such blood pump 40 is, for example, a tube pump.

The dialysate circuit 14 includes, for example, a dialysate supply circuit 50 leading from a dialysate supply source (not shown) to the liquid port 10c of the blood purifier 10, and a dialysate discharge circuit 51 leading from the liquid port 10d of the blood purifier 10 to the outside of the apparatus. The dialysate circuit 14 is provided with, for example, a dialysate supply pump 52 and a dialysate discharge pump 53.

The citric acid solution supply means 12 includes, for example, a solution reservoir 60 in which a citric acid solution is stored, a connecting line 61 that connects the solution reservoir 60 and the blood collection circuit 31, and a pump 62 for supplying the citric acid solution in the solution reservoir 60 into the blood collection circuit 31.

The calcium solution supply means 13 includes, for example, a solution reservoir 70 in which a calcium solution is stored, a connecting line 71 that connects the solution reservoir 70 and the blood return circuit 33, and a pump 72 for supplying the calcium solution in the solution reservoir 70 into the blood return circuit 33.

The replacement fluid circuit 15 includes, for example, a replacement fluid supply source 80, a replacement fluid line 81 that connects the replacement fluid supply source 80 and the blood return circuit 33, and a replacement fluid pump 82 for supplying the replacement fluid in the replacement fluid supply source 80 into the blood return circuit 33.

The control device 16 is, for example, a microcomputer having a CPU, a memory, and the like. The control device 16 is able to carry out blood purification treatment by controlling the operation of the devices, including, for example, the blood pump 40, dialysate supply pump 52, dialysate discharge pump 53, replacement fluid pump 82, citric acid solution supply means 12 and calcium solution supply means 13. The control device 16 can implement blood purification treatment by, for example, executing a program stored in a memory in advance.

For example, the control device 16 estimates, based on an input of a target calcium ion concentration for the patient's blood, an amount of calcium loss occurring in the blood circuit 11 as a whole, including an amount of calcium removed by the blood purifier 10 when blood having the target calcium ion concentration flows through the blood circuit 11. The control device 16 then calculates the flow rate of the calcium solution to be supplied to the blood circuit 11 based on the estimated amount of calcium loss and, based on the obtained flow rate of the calcium solution, supplies the calcium solution into the blood circuit 11 by the calcium solution supply means 13.

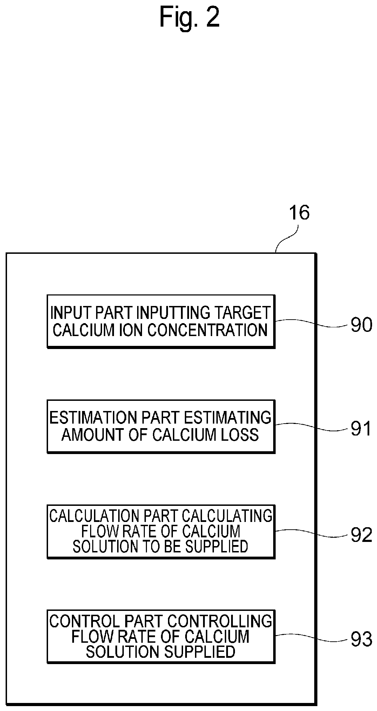

As illustrated in Fig. 2, the control device 16 includes, for example: an input part 90 that inputs a target calcium ion concentration for the patient's blood; an estimation part 91 that estimates, based on an input of a target calcium ion concentration for the patient's blood, an amount of calcium loss occurring in the blood circuit 11 as a whole, including an amount of calcium removed by the blood purifier 10 when blood having the target calcium ion concentration flows through the blood circuit 11; a calculation part 92 that calculates the flow rate of the calcium solution to be supplied to the blood circuit 11 based on the estimated amount of calcium loss; and a control part 93 that supplies the calcium solution to the blood circuit 11 through the calcium solution supply means 13 based on the calculated flow rate of the calcium solution.

The control device 16 calculates the flow rate of the calcium solution (Qca) to be supplied to the blood circuit 11 by the calcium solution supply means 13 according to the following expression (1):

$$Qca = \{\{(Qd*kd+Qf*kf)*Ctca*kca-Qr*Crca\}/Cica\} \ \dots \ (1)$$

wherein:

Ctca is a target calcium ion concentration for a patient's blood;

Cica is a concentration of a calcium solution in the calcium solution supply means;

kca is a constant used for calculating the concentration of calcium that can be subjected to dialysis and/or filtration at the blood purifier, based on the target calcium ion concentration Ctca;

Qd is a flow rate of dialysate passing through the blood purifier;

kd is a constant used for calculating the amount of calcium removed by dialysis based on the flow rate of dialysate Qd;

Qf is a flow rate of filtration at the blood purifier;

kf is a constant used for calculating the amount of calcium removed by filtration based on the flow rate of filtration Qf;

Qr is a flow rate of a replacement fluid added to the blood circuit; and

Crca is a calcium concentration in the replacement fluid.

In the blood circuit 11, the amount of calcium removed at the blood purifier 10 when blood having a target calcium ion concentration flows through the blood circuit 11 can be obtained by (Qd*kd+Qf*kf)*Ctca*kca. Further, the amount of calcium that increases in the blood circuit 11 due to the replacement fluid can be obtained by Qr*Crca. Accordingly, the amount of calcium loss in the entire blood circuit 11 can be obtained by (Qd*kd+Qf*kf)*Ctca*kca-Qr*Crca. Then, the obtained calcium loss in the entire blood circuit 11 ((Qd*kd+Qf*kf)*Ctca*kca-Qr*Crca) divided by the concentration of the calcium solution in the calcium solution supply means 13 will be the flow rate of the calcium solution (Qca) to be supplied by the calcium solution supply means 13. The present embodiment may be configured such that no replacement fluid is added in the blood purification treatment; and, in that case, the flow rate of a replacement fluid Qr will be zero.

An example of the blood purification treatment using the blood purification apparatus 1 will now be described.

First, a target calcium ion concentration for the patient's blood is entered before the start of the blood purification treatment. The target calcium ion concentration is determined by a medical service worker according to the patient's condition, etc. Next, the needles of the blood collection part 30 and the blood return part 32 are inserted into the patient, and the blood purification treatment is started. The blood pump 40 of the blood circuit 11 is activated so that the patient's blood is delivered through the blood collection circuit 31 to the primary side of the blood purification membrane 20 of the blood purifier 10, and the blood, after passing through the primary side of the blood purification membrane 20, is returned to the patient through the blood return circuit 33.

In the dialysate circuit 14, with the aid of the dialysate supply pump 52, dialysate discharge pump 53, etc., dialysate is supplied to the secondary side of the blood purification membrane 20 of the blood purifier 10 through the dialysate supply circuit 50 and, after the passage through the secondary side of the blood purification membrane 20, the dialysate is discharged through the dialysate discharge circuit 51.

A predetermined flow rate of citric acid solution is supplied by the citric acid solution supply means 12 into the blood flowing from the blood collection part 30 toward the blood purifier 10. With such supply of citric acid solution, the calcium ion concentration in the blood decreases so that blood coagulation is suppressed. When the blood passes through the blood purifier 10, some components in the blood, including calcium ions, pass through the blood purification membrane 20 with the effect of filtration by the blood purification membrane 20 and the effect of osmotic pressure given by the dialysate flowing in the secondary side of the blood purification membrane 20, and such components are discharged into the dialysate discharge circuit 51 together with the dialysate.

A predetermined flow rate of replacement fluid is supplied from the replacement fluid circuit 15 to the blood flowing from the blood purifier 10 toward the blood return part 32. As a result, the patient's electrolytes, etc., can be recovered.

Furthermore, a predetermined flow rate of calcium solution is supplied by the calcium solution supply means 13 into the blood flowing from the blood purifier 10 toward the blood return part 32. As a result, the calcium concentration in the blood can be recovered. After that, the blood is returned to the patient via the blood return part 32.

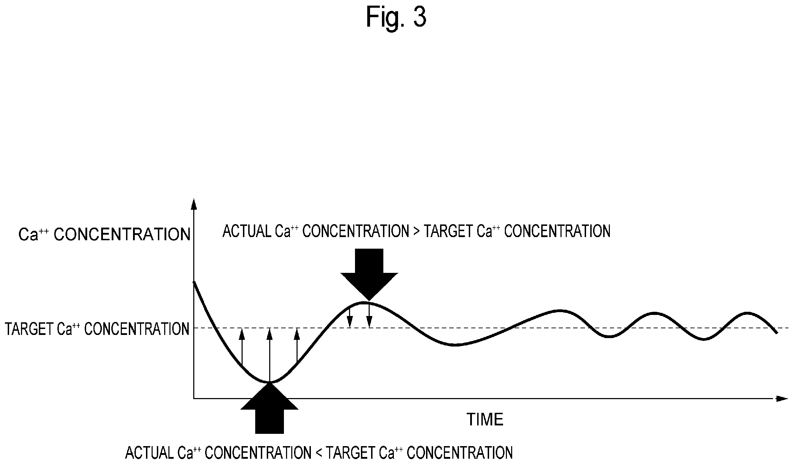

The flow rate of the calcium solution supplied to the patient's blood is a flow rate calculated according to the expression (1) based on the target calcium ion concentration. In this calculation, the amount of calcium loss in the entire blood circuit 11 is estimated by taking into account: (i) the amount of calcium removed by the blood purifier 10 when blood having the target calcium ion concentration flows through the blood circuit 11; and (ii) the amount of calcium increase caused by the addition of the replacement fluid, and, based on the estimated calcium loss, the flow rate of the calcium solution to be supplied is determined. Accordingly, as shown in Fig. 3, if the actual calcium ion concentration in the patient's blood is lower than the target calcium ion concentration, the amount of calcium ions added to the blood will be greater than the necessary amount of calcium ions to be added. When the treatment is continued in this state, the calcium ion concentration in the blood gradually increases. When the calcium ion concentration in the blood continues to increase and the actual calcium ion concentration in the blood exceeds the target calcium ion concentration, then the amount of calcium ions added to the blood becomes smaller than the necessary amount of calcium ions to be added, resulting in the calcium ion concentration in the blood gradually decreasing. When the above process is repeated, the actual calcium ion concentration in the blood gradually approaches, and then meets, the target calcium ion concentration.

According to the present embodiment: the amount of calcium loss throughout the blood circuit 11 is estimated based on an input of a target calcium ion concentration for the patient's blood, such amount of calcium loss including the amount of calcium removed by the blood purifier 10 when blood having the target calcium ion concentration flows through the blood circuit 11; the flow rate of the calcium solution to be supplied to the blood circuit 11 is calculated based on the estimated amount of calcium loss; and the calcium solution is supplied to the blood circuit 11 by the calcium solution supply means 13 based on the calculated flow rate of the calcium solution. With this configuration, the calcium ion concentration in the blood automatically approaches the target calcium ion concentration. Accordingly, during the purification of the patient's blood, the calcium concentration in the blood can be adjusted in a simple and automatic manner.

Second Embodiment

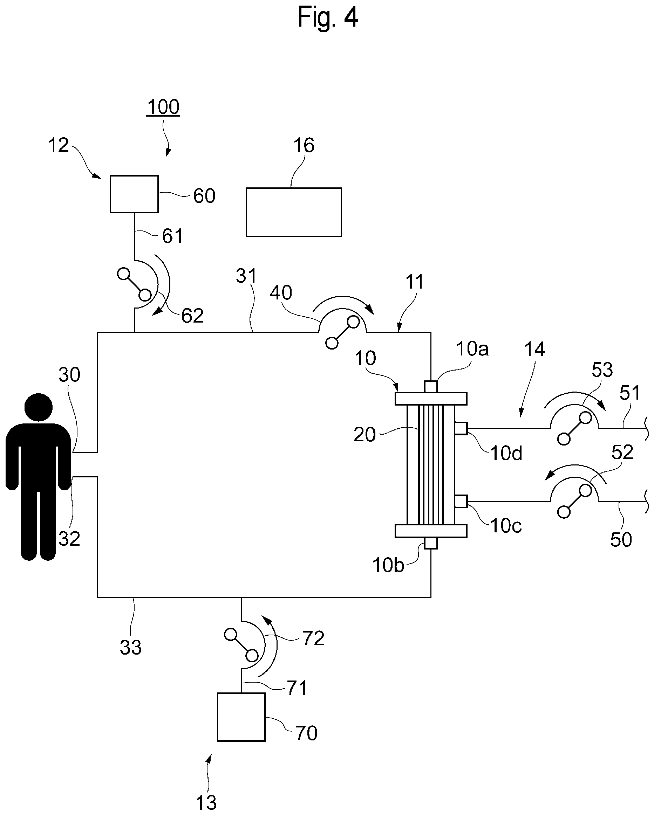

Next, an example in which the present invention is applied to a blood purification apparatus 100 for blood dialysis (hemodialysis) will be described. Fig. 4 is an illustration showing the schematic configuration of the blood purification apparatus 100 according to the present embodiment.

When compared to the blood purification apparatus 1 of the first embodiment, the blood purification apparatus 100 is configured to, for example, have no replacement fluid circuit 15.

The control device 16 calculates the flow rate of the calcium solution (Qca) to be supplied to the blood circuit 11 by the calcium solution supply means 13 according to the following expression (2):

$$Qca = \{\{(Qd*kd+Qf*kf)*Ctca*kca\}/Cica\} \dots (2)$$

wherein:

Ctca is a target calcium ion concentration for a patient's blood;

Cica is a concentration of a calcium solution in the calcium solution supply means;

kca is a constant used for calculating the concentration of calcium that can be subjected to dialysis and/or filtration at the blood purifier, based on the target calcium ion concentration Ctca;

Qd is a flow rate of dialysate passing through the blood purifier;

kd is a constant used for calculating the amount of calcium removed by dialysis based on the flow rate of dialysate Qd;

Qf is a flow rate of filtration at the blood purifier; and

kf is a constant used for calculating the amount of calcium removed by filtration based on the flow rate of filtration Qf.

In the present embodiment, the amount of calcium removed at the blood purifier 10 when blood having a target calcium ion concentration flows through the blood circuit 11 can be obtained by (Qd*kd+Qf*kf)*Ctca*kca. Further, unlike the first embodiment, the present embodiment does not have any increase of calcium due to the addition of a replacement fluid. Thus, the amount of calcium loss in the entire blood circuit 11 can be obtained by (Qd*kd+Qf*kf)*Ctca*kca. Then, the obtained calcium loss ((Qd*kd+Qf*kf)*Ctca*kca) divided by the concentration of the calcium solution in the calcium solution supply means 13 will be the flow rate of the calcium solution (Qca) to be supplied by the calcium solution supply means 13.

Since other portions of the configuration of the present embodiment are the same as those in the first embodiment, the same reference numerals will be used for such other portions and descriptions thereof will be omitted.

According to the present embodiment: the amount of calcium loss throughout the blood circuit 11 is estimated based on an input of a target calcium ion concentration for the patient's blood, such amount of calcium loss including the amount of calcium removed by the blood purifier 10 when blood having the target calcium ion concentration flows through the blood circuit 11; the flow rate of the calcium solution to be supplied to the blood circuit 11 is calculated based on the estimated amount of calcium loss; and the calcium solution is supplied to the blood circuit 11 by the calcium solution supply means 13 based on the calculated flow rate of the calcium solution. With this configuration, the calcium ion concentration in the blood automatically approaches the target calcium ion concentration, as shown in Fig. 3. Accordingly, during the purification of the patient's blood, the calcium concentration in the blood can be adjusted in a simple and automatic manner.

Third Embodiment

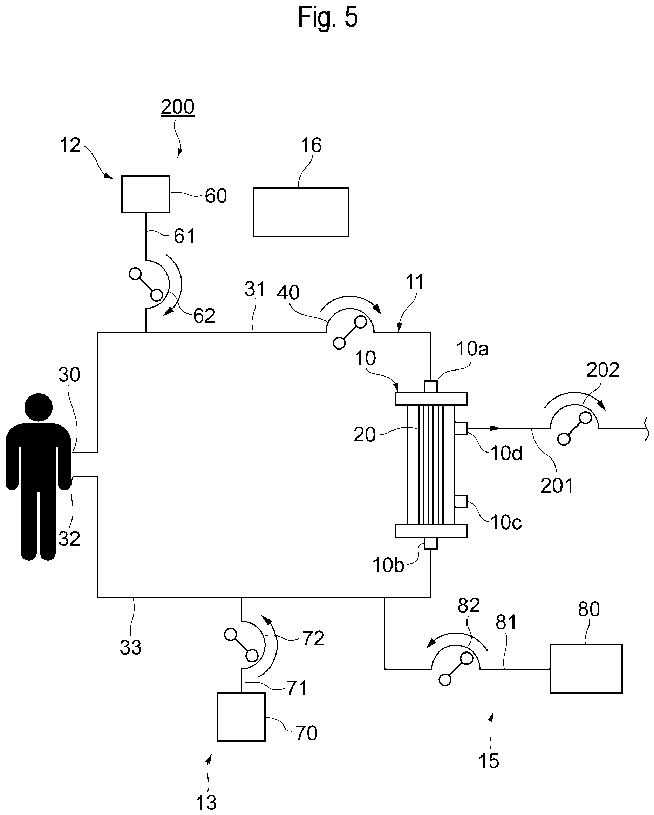

Next, an example in which the present invention is applied to a blood purification apparatus 200 for filtration of blood (hemofiltration) will be described. Fig. 5 is an illustration showing the schematic configuration of the blood purification apparatus 200 according to the present embodiment.

When compared to the blood purification apparatus 1 of the first embodiment, the blood purification apparatus 200 is configured to, for example, have no dialysate circuit 14 but instead have a discharge circuit 201.

For example, the discharge circuit 201 leads from the liquid port 10d of the blood purifier 10 to a discharge part, which is external to the apparatus. The discharge circuit 201 is provided with a discharge pump 202. The liquid port 10c is closed.

The control device 16 calculates the flow rate of the calcium solution (Qca) to be supplied to the blood circuit 11 by the calcium solution supply means 13 according to the following expression (3):

$$Qca = \{(Qf*kf*Ctca*kca-Qr*Crca)/Cica\} \dots (3)$$

wherein:

Ctca is a target calcium ion concentration for a patient's blood;

Cica is a concentration of a calcium solution in the calcium solution supply means;

kca is a constant used for calculating the concentration of calcium that can be subjected to filtration at the blood purifier, based on the target calcium ion concentration Ctca;

Qf is a flow rate of filtration at the blood purifier;

kf is a constant used for calculating the amount of calcium removed by filtration based on the flow rate of filtration Qf;

Qr is a flow rate of a replacement fluid added to the blood circuit; and

Crca is a calcium concentration in the replacement fluid.

In the present embodiment, the amount of calcium removed at the blood purifier 10 when blood having a target calcium ion concentration flows through the blood circuit 11 can be obtained by Qf*kf*Ctca*kca. Further, the amount of calcium that increases in the blood circuit 11 due to the replacement fluid can be obtained by Qr*Crca. Accordingly, the amount of calcium loss in the entire blood circuit 11 can be obtained by (Qf*kf*Ctca*kca-Qr*Crca). Then, the obtained calcium loss (Qf*kf*Ctca*kca-Qr*Crca) divided by the concentration of the calcium solution in the calcium solution supply means 13 will be the flow rate of the calcium solution (Qca) to be supplied by the calcium solution supply means 13. The present embodiment may be configured such that no replacement fluid is added in the blood purification treatment and, in that case, the flow rate of a replacement fluid Qr will be zero.

Since other portions of the configuration of the present embodiment are the same as those in the above-described embodiments, the same reference numerals will be used for such other portions and descriptions thereof will be omitted.

According to the present embodiment: the amount of calcium loss throughout the blood circuit 11 is estimated based on an input of a target calcium ion concentration for the patient's blood, such amount of calcium loss including the amount of calcium removed by the blood purifier 10 when blood having the target calcium ion concentration flows through the blood circuit 11; the flow rate of the calcium solution to be supplied to the blood circuit 11 is calculated based on the estimated amount of calcium loss; and the calcium solution is supplied to the blood circuit 11 by the calcium solution supply means 13 based on the calculated flow rate of the calcium solution. With this configuration, the calcium ion concentration in the blood automatically approaches the target calcium ion concentration, as shown in Fig. 3. Accordingly, during the purification of the patient's blood, the calcium concentration in the blood can be adjusted in a simple and automatic manner.

The first to third embodiments described above may be configured such that users can correct the flow rate of the calcium solution to be supplied. For example, the blood purification apparatus 1 may be configured so as to be able to set a correction term for correcting the flow rate of the calcium solution. For example, the blood purification apparatus 1 may have an input part that inputs a correction term for the flow rate of the calcium solution to be supplied.

Specifically, the flow rate of the calcium solution (Qca) to be supplied according to the first embodiment is calculated by the following expression (1)', using a correction term set by a user:

$$Qca = \{\{(Qd*kd+Qf*kf)*Ctca*kca-Qr*Crca+Qeff*Cadca\}/Cica\} \dots (1)'$$

wherein Cadca is a correction term set by a user.

The flow rate of the calcium solution (Qca) to be supplied according to the second embodiment is calculated by the following expression (2)':

$$Qca = \{\{(Qd*kd+Qf*kf)*Ctca*kca+Qeff*Cadca\}/Cica\} \dots (2)'$$

wherein Cadca is a correction term set by a user.

The flow rate of the calcium solution (Qca) to be supplied according to the third embodiment is calculated by the following expression (3)':

$$Qca = \{(Qd*kd*Ctca*kca-Qr*Crca+Qeff*Cadca\}/Cica\} \dots (3)'$$

wherein Cadca is a correction term set by a user.

It should be noted here that Ctca may represent the concentration of calcium ions in the patient's blood or the concentration of calcium, including calcium ions, in the patient's blood. In that case, kca is a constant used for calculating the concentration of calcium that can be subjected to filtration at the blood purifier, based on such Ctca being the patient's calcium ion concentration or calcium concentration.

In the above-described examples, if, for example, the patient's calcium ion concentration is distant from the target calcium ion concentration, the calcium concentration of the patient can be adjusted more accurately and quickly with such correction term set by a user.

Preferred embodiments of the present invention have been described above with reference to the attached drawings.

In the above-described first to third embodiments, the flow rate of the calcium solution to be supplied is calculated according to expressions (1) to (3), but the expressions are not limited thereto. The circuit configuration of each of the blood purification apparatuses 1, 100 and 200 is not limited to the configuration described in those embodiments. The blood purifier may have blood purification membranes other than hollow fiber membranes. The present invention can also be applied to blood purification apparatuses for continuous hemofiltration, continuous hemodialysis, continuous hemodiafiltration, SCUF (slow continuous ultrafiltration), etc.

Industrial Applicability

The present invention is useful in providing blood purification apparatuses capable of adjusting the concentration of calcium in a patient's blood in a simple and automatic manner during the purification of the patient's blood.

Reference Signs List

1      Blood Purification Apparatus

10     Blood Purifier

11     Blood Circuit

12     Citric Acid Solution Supply Means

13     Calcium Solution Supply Means

14     Dialysate Circuit

15     Replacement Fluid Circuit

16     Control Device

20     Blood Purification Membrane

40     Blood Pump

**Claims**

1.  A blood purification apparatus comprising:

    a blood circuit (11);
    a blood purifier (10) provided in the blood circuit; citric acid solution supply means (12) that supplies a citric acid solution into the blood circuit upstream of the blood purifier; and
    calcium solution supply means (13) that supplies a calcium solution into the blood circuit downstream of the blood purifier,
    **characterized in that** the blood purification apparatus is configured to:

    estimate, based on an input of a target calcium ion concentration for a patient's blood, an amount of calcium loss throughout the blood circuit, including an amount of calcium removed by the blood purifier when blood having the target calcium ion concentration flows through the blood circuit;
    calculate a flow rate of the calcium solution to be supplied to the blood circuit based on the estimated amount of calcium loss; and
    supply the calcium solution to the blood circuit by the calcium solution supply means based on the calculated flow rate of the calcium solution.

2.  The blood purification apparatus according to claim 1, wherein the flow rate of the calcium solution (Qca) to be supplied to the blood circuit by the calcium solution supply means is calculated according to:

$$Qca = \{\{(Qd*kd+Qf*kf)*Ctca*kca-Qr*Crca\}/Cica\}$$

wherein:

Ctca is a target calcium ion concentration for the patient's blood;
Cica is a concentration of the calcium solution in the calcium solution supply means;
kca is a constant used for calculating a concentration of calcium that can be subjected to dialysis and/or filtration at the blood purifier based on the target calcium ion concentration Ctca;
Qd is a flow rate of dialysate passing through the blood purifier;
kd is a constant used for calculating an amount of calcium removed by dialysis based on the flow rate of dialysate Qd;
Qf is a flow rate of filtration at the blood purifier;
kf is a constant used for calculating an amount of calcium removed by filtration based on the flow rate of filtration Qf;
Qr is a flow rate of a replacement fluid added to the blood circuit; and
Crca is a calcium concentration of the replacement fluid.

3. The blood purification apparatus according to claim 1, wherein the flow rate of the calcium solution (Qca) to be supplied to the blood circuit by the calcium solution supply means is calculated according to:

$$Qca = \{\{(Qd*kd+Qf*kf)*Ctca*kca\}/Cica\}$$

wherein:

Ctca is a target calcium ion concentration for the patient's blood;
Cica is a concentration of the calcium solution in the calcium solution supply means;
kca is a constant used for calculating a concentration of calcium that can be subjected to dialysis and/or filtration at the blood purifier based on the target calcium ion concentration Ctca;
Qd is a flow rate of dialysate passing through the blood purifier;
kd is a constant used for calculating an amount of calcium removed by dialysis based on the flow rate of dialysate Qd;
Qf is a flow rate of filtration at the blood purifier; and
kf is a constant used for calculating an amount of calcium removed by filtration based on the flow rate of filtration Qf.

4. The blood purification apparatus according to claim 1, wherein the flow rate of the calcium solution (Qca) to be supplied to the blood circuit by the calcium solution supply means is calculated according to:

$$Qca = \{(Qf*kf*Ctca*kca-Qr*Crca\}/Cica\}$$

wherein:

Ctca is a target calcium ion concentration for the patient's blood;
Cica is a concentration of the calcium solution in the calcium solution supply means;
kca is a constant used for calculating a concentration of calcium that can be subjected to filtration at the blood purifier based on the target calcium ion concentration Ctca;
Qf is a flow rate of filtration at the blood purifier;
kf is a constant used for calculating an amount of calcium removed by filtration based on the flow rate of filtration Qf;
Qr is a flow rate of a replacement fluid added to the blood circuit; and
Crca is a calcium concentration of the replacement fluid.

5. The blood purification apparatus according to any one of claims 2 to 4, wherein a correction term can be set to correct the flow rate of the calcium solution to be supplied.

**Patentansprüche**

1. Blutreinigungsvorrichtung, umfassend:

   einen Blutkreislauf (11);
   einen Blutreiniger (10), der in dem Blutkreislauf angeordnet ist;
   eine Zitronensäurelösungszufuhreinrichtung (12), die stromaufwärts des Blutreinigers eine Zitronensäurelösung in den Blutkreislauf zuführt; und
   eine Calciumlösungszufuhreinrichtung (13), die stromabwärts des Blutreinigers eine Calciumlösung in den Blutkreislauf zuführt;
   **dadurch gekennzeichnet, dass** die Blutreinigungsvorrichtung so konfiguriert ist, dass sie:

   auf der Basis einer Eingabe einer Calciumionen-Sollkonzentration für das Blut eines Patienten eine Menge des Calciumverlusts im gesamten Blutkreislauf schätzen kann, einschließlich einer Menge an Calcium, die durch den Blutreiniger entfernt wird, wenn Blut mit der Calciumionen-Sollkonzentration durch den Blutkreislauf fließt;
   auf der Basis der geschätzten Menge des Calciumverlusts eine Fließgeschwindigkeit der dem Blutkreislauf zuzuführenden Calciumlösung berechnen kann; und
   auf der Basis der berechneten Fließgeschwindigkeit der Calciumlösung dem Blutkreislauf die Calciumlösung durch die Calciumlösungszufuhreinrichtung zuführen kann.

2. Blutreinigungsvorrichtung gemäß Anspruch 1, wobei die Fließgeschwindigkeit der durch die Calciumlösungszufuhreinrichtung dem Blutkreislauf zuzuführenden Calciumlösung (Qca) berechnet wird gemäß

$$Qca = \{\{(Qd*kd+Qf*kf)*Ctca*kca-Qr*Crca\}/Cica\}$$

   wobei:

   Ctca eine Calciumionen-Sollkonzentration für das Blut des Patienten ist;
   Cica eine Konzentration der Calciumlösung in der Calciumlösungszufuhreinrichtung ist;
   kca eine Konstante ist, die verwendet wird, um auf der Basis der Calciumionen-Sollkonzentration Ctca eine Konzentration von Calcium zu berechnen, das einer Dialyse und/oder Filtration am Blutreiniger unterzogen werden kann;
   Qd eine Fließgeschwindigkeit von durch den Blutreiniger fließendem Dialysat ist;
   kd eine Konstante ist, die verwendet wird, um auf der Basis der Fließgeschwindigkeit des Dialysats Qd die Menge des durch die Dialyse entfernten Calciums zu berechnen;
   Qf eine Fließgeschwindigkeit der Filtration am Blutreiniger ist;
   kf eine Konstante ist, die verwendet wird, um auf der Basis der Fließgeschwindigkeit der Filtration Qf die Menge des durch die Filtration entfernten Calciums zu berechnen;
   Qr eine Fließgeschwindigkeit einer dem Blutkreislauf hinzugefügten Ersatzflüssigkeit ist; und
   Crca die Calciumkonzentration der Ersatzflüssigkeit ist.

3. Blutreinigungsvorrichtung gemäß Anspruch 1, wobei die Fließgeschwindigkeit der durch die Calciumlösungszufuhreinrichtung dem Blutkreislauf zuzuführenden Calciumlösung (Qca) berechnet wird gemäß

$$Qca = \{\{(Qd*kd+Qf*kf)*Ctca*kca\}/Cica\}$$

   wobei:

   Ctca eine Calciumionen-Sollkonzentration für das Blut des Patienten ist;
   Cica eine Konzentration der Calciumlösung in der Calciumlösungszufuhreinrichtung ist;
   kca eine Konstante ist, die verwendet wird, um auf der Basis der Calciumionen-Sollkonzentration Ctca eine Konzentration von Calcium zu berechnen, das einer Dialyse und/oder Filtration am Blutreiniger unterzogen werden kann;
   Qd eine Fließgeschwindigkeit von durch den Blutreiniger fließendem Dialysat ist;

kd eine Konstante ist, die verwendet wird, um auf der Basis der Fließgeschwindigkeit des Dialysats Qd die Menge des durch die Dialyse entfernten Calciums zu berechnen;

Qf eine Fließgeschwindigkeit der Filtration am Blutreiniger ist; und

kf eine Konstante ist, die verwendet wird, um auf der Basis der Fließgeschwindigkeit der Filtration Qf die Menge des durch die Filtration entfernten Calciums zu berechnen.

4.  Blutreinigungsvorrichtung gemäß Anspruch 1, wobei die Fließgeschwindigkeit der durch die Calciumlösungszufuhreinrichtung dem Blutkreislauf zuzuführenden Calciumlösung (Qca) berechnet wird gemäß

$$Qca = \{(Qf*kf*Ctca*kca-Qr*Crca\}/Cica\}$$

wobei:

Ctca eine Calciumionen-Sollkonzentration für das Blut des Patienten ist;

Cica eine Konzentration der Calciumlösung in der Calciumlösungszufuhreinrichtung ist;

kca eine Konstante ist, die verwendet wird, um auf der Basis der Calciumionen-Sollkonzentration Ctca eine Konzentration von Calcium zu berechnen, das einer Dialyse und/oder Filtration am Blutreiniger unterzogen werden kann;

Qf eine Fließgeschwindigkeit der Filtration am Blutreiniger ist;

kf eine Konstante ist, die verwendet wird, um auf der Basis der Fließgeschwindigkeit der Filtration Qf die Menge des durch die Filtration entfernten Calciums zu berechnen;

Qr eine Fließgeschwindigkeit einer dem Blutkreislauf hinzugefügten Ersatzflüssigkeit ist; und

Crca die Calciumkonzentration der Ersatzflüssigkeit ist.

5.  Blutreinigungsvorrichtung gemäß einem der Ansprüche 2 bis 4, wobei ein Korrekturterm eingestellt werden kann, um die Fließgeschwindigkeit der zuzuführenden Calciumlösung zu korrigieren.

**Revendications**

1.  Appareil de purification du sang comprenant:

un circuit de sang (11);

un purificateur de sang (10) prévu dans le circuit de sang;

un moyen de fourniture de solution d'acide citrique (12) qui fournit une solution d'acide citrique dans le circuit de sang en amont du purificateur de sang; et

un moyen de fourniture de solution de calcium (13) qui fournit une solution de calcium dans le circuit de sang en aval du purificateur de sang,

**caractérisé en ce que** l'appareil de purification du sang est configuré pour:

estimer, sur la base d'une introduction d'une concentration d'ions calcium cible pour le sang d'un patient, une quantité de perte de calcium dans tout le circuit de sang, incluant une quantité de calcium retirée par le purificateur de sang lorsque du sang ayant la concentration d'ions calcium cible circule dans le circuit de sang;

calculer un débit de la solution de calcium destinée à être fournie au circuit de sang sur la base de la quantité estimée de perte de calcium; et

fournir la solution de calcium au circuit de sang par le moyen de fourniture de solution de calcium sur la base du débit calculé de la solution de calcium.

2.  Appareil de purification du sang selon la revendication 1, dans lequel le débit de la solution de calcium (Qca) destinée à être fournie au circuit de sang par le moyen de fourniture de solution de calcium est calculé selon:

$$Qca = \{\{(Qd*kd+Qf*kf)*Ctca*kca-Qr*Crca\}/Cica\}$$

ou:

Ctca est une concentration d'ions calcium cible pour le sang du patient;
Cica est une concentration de la solution de calcium dans le moyen de fourniture de solution de calcium;
kca est une constante utilisée pour calculer une concentration de calcium qui peut être soumise à une dialyse et/ou une filtration au niveau du purificateur de sang sur la base de la concentration d'ions calcium cible Ctca;
Qd est un débit de dialysat traversant le purificateur de sang;
kd est une constante utilisée pour calculer une quantité de calcium retirée par dialyse sur la base du débit de dialysat Qd;
Qf est un débit de filtration au niveau du purificateur de sang;
kf est une constante utilisée pour calculer une quantité de calcium retirée par filtration sur la base du débit de filtration Qf;
Qr est un débit d'un fluide de remplacement ajouté au circuit de sang; et
Crca est une concentration de calcium du fluide de remplacement.

3. Appareil de purification du sang selon la revendication 1, dans lequel le débit de la solution de calcium (Qca) destinée à être fournie au circuit de sang par le moyen de fourniture de solution de calcium est calculé selon:

$$Qca = \{\{(Qd*kd+Qf*kf)*Ctca*kca\}/Cica\}$$

dans laquelle:

Ctca est une concentration d'ions calcium cible pour le sang du patient;
Cica est une concentration de la solution de calcium dans le moyen de fourniture de solution de calcium;
kca est une constante utilisée pour calculer une concentration de calcium qui peut être soumise à une dialyse et/ou une filtration au niveau du purificateur de sang sur la base de la concentration d'ions calcium cible Ctca;
Qd est un débit de dialysat traversant le purificateur de sang;
kd est une constante utilisée pour calculer une quantité de calcium retirée par dialyse sur la base du débit de dialysat Qd;
Qf est un débit de filtration au niveau du purificateur de sang; et
kf est une constante utilisée pour calculer une quantité de calcium retirée par filtration sur la base du débit de filtration Qf.

4. Appareil de purification du sang selon la revendication 1, dans lequel le débit de la solution de calcium (Qca) destinée à être fournie au circuit de sang par le moyen de fourniture de solution de calcium est calculé selon:

$$Qca = \{(Qf*kf*Ctca*kca-Qr*Crca\}/Cica\}$$

où:

Ctca est une concentration d'ions calcium cible pour le sang du patient;
Cica est une concentration de la solution de calcium dans le moyen de fourniture de solution de calcium;
kca est une constante utilisée pour calculer une concentration de calcium qui peut être soumise à une filtration au niveau du purificateur de sang sur la base de la concentration d'ions calcium cible Ctca;
Qf est un débit de filtration au niveau du purificateur de sang;
kf est une constante utilisée pour calculer une quantité de calcium retirée par filtration sur la base du débit de filtration Qf;
Qr est un débit d'un fluide de remplacement ajouté au circuit de sang; et
Crca est une concentration de calcium du fluide de remplacement.

5. Appareil de purification du sang selon l'une quelconque des revendications 2 à 4, dans lequel un terme de correction peut être établi pour corriger le débit de la solution de calcium destinée à être fournie.

# Fig. 1

# Fig. 2

16

```
┌─────────────────────────────────────┐
│                                      │
│  ┌────────────────────────────┐      │
│  │ INPUT PART INPUTTING TARGET│──── 90│
│  │ CALCIUM ION CONCENTRATION  │      │
│  └────────────────────────────┘      │
│                                      │
│  ┌────────────────────────────┐      │
│  │ ESTIMATION PART ESTIMATING │──── 91│
│  │ AMOUNT OF CALCIUM LOSS     │      │
│  └────────────────────────────┘      │
│                                      │
│  ┌────────────────────────────┐      │
│  │CALCULATION PART CALCULATING│──── 92│
│  │   FLOW RATE OF CALCIUM     │      │
│  │   SOLUTION TO BE SUPPLIED  │      │
│  └────────────────────────────┘      │
│                                      │
│  ┌────────────────────────────┐      │
│  │ CONTROL PART CONTROLLING   │──── 93│
│  │   FLOW RATE OF CALCIUM     │      │
│  │   SOLUTION SUPPLIED        │      │
│  └────────────────────────────┘      │
│                                      │
└─────────────────────────────────────┘
```

Fig. 3

# Fig. 4

# Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9039647 B
- US 2014353251 A
- US 20160121035 A